# EUROPEAN PATENT APPLICATION

(11) **EP 1 547 649 A1**
(43) Date of publication of application: **29.06.2005**
(21) Application number: 03700528.7
(22) Date of filing: 10.01.2003
(51) Int. Cl.: A61N 1/10

(54) **POTENTIAL THERAPEUTIC DEVICE**

(30) Priority: 01.10.2002 JP 2002288174
(71) Applicant: Japan Electronics Science R & D Co., Ltd., Utsunomiya-shi, Tochigi 321-0945 (JP)
(72) Inventor: OHASHI, Keiichi, Japan Elec. Science R&D Co., Ltd., Utsunomiya-shi, Tochigi 321-0945 (JP); TAKIZAWA, Shinjiro Japan Elec. Sc. R&D Co., Ltd., Utsunomiga-shi, Tochigi 321-0945 (JP)
(74) Representative: Brown, Kenneth Richard
(86) International application number: PCT/JP2003/000164
(87) International publication number: WO 2004/030754

(57) **Abstract**

An electric potential therapy apparatus has been prepared with a plurality of alternating current patterns, made by combining varying voltages, waveforms and frequencies with the passage of time. The user selects one from the alternating patterns in accordance with the user's physical constitution and physical condition, so that an a.c. generating circuit (9) generates a high voltage alternating current which changes its voltage, waveform and frequency in accordance with the alternating current pattern, to be applied to an electrode (1). A massage unit (11) having kneading rollers moving up and down is arranged in a backrest (10) of the electric potential therapy apparatus while a roller massager is provided for a footrest (14). Further, an ion generating unit (16) is provided over the backrest (10).

## Description

### Technical Field

The present invention relates to an electric potential therapy apparatus having effect on stiff shoulders, headache, insomnia, chronic constipation, etc., and furthermore relates to an electric potential therapy apparatus equipped with a massage machine and/or an ion generator.

### Background Art

It has been known that electric potential therapy apparatuses which give treatment by applying negative high-voltage electric fields have effect on stiff shoulders, headache, insomnia, chronic constipation, etc. This treatment, however, does not present its effect for all the people with these symptoms, and may have significant effect for some, less effect for others.

Alternatively, in some cases, people who have experienced benefits the first time, but may receive diminished effect or no longer any effect as they repeat it a few times or more. On the contrary, there are other cases where people who receive no effect at the first time, but can experience increased effect as they repeat it a few times and more.

Further, some people may have some other symptoms from which they did not suffer before due to an improvement in their condition when they use it.

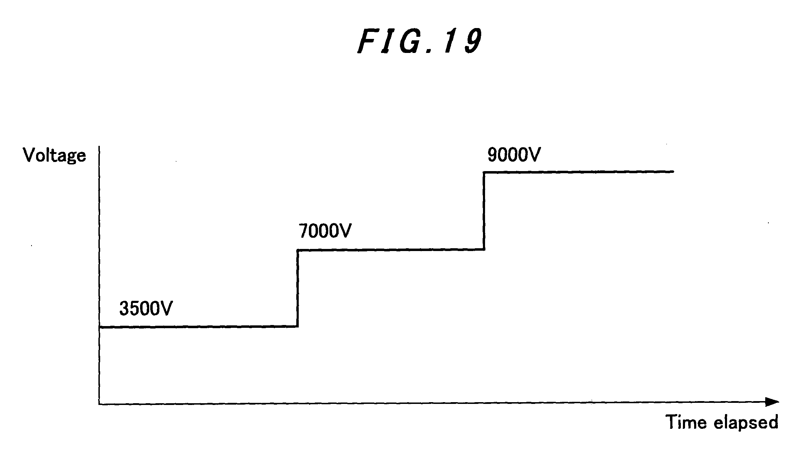

As stated above, every person has a different reaction to the electric potential therapy apparatus, and even an identical person may experience greatly different physical reactions and effects depending on the number of use, due to getting used to, and/or due to their physical condition at different points in time. One of the considered causes of the above situations is that the conventional electric potential therapy apparatus gives treatment to different users of different physical constitutions and physical conditions, in a like manner, by changing the voltage for generation of the electric field in a constant fixed pattern, as shown in FIG. 19.

There is also a proposal that the voltage can be changed with the passage of therapy time (see a patent literature 1, for example)

### [Patent literature 1]

Japanese Patent Application Laid-open 2000-189525

Changing the voltage with the passage of therapy time is not a change aiming at meeting the physical constitution and physical condition of an individual but is merely an attempt to prevent conditioning.

It is therefore an object of the present invention to provide an electric potential therapy apparatus capable of providing therapy of the most suitable intensities depending on various users of different physical constitutions and physical conditions or depending on the degree of the identical user's conditioning to the electric potential therapy apparatus. It is another object of the present invention to provide an electric potential therapy apparatus which can produce improved effect.

### Disclosure of Invention

In order to attain the above objects, according to the first aspect of the present invention, an electric potential therapy apparatus for generating a negative electric field by applying a high-voltage alternating current that is biased to a negative side, to an electric field generating plate electrode, includes: a voltage change pattern storing means for storing a plurality of voltage change patterns on which a voltage is changed with time; a voltage change pattern selecting means for selecting one from the plurality of voltage change patterns stored in the voltage change pattern storing means; and an alternating current generating circuit which reads the voltage change pattern selected by the voltage change pattern selecting means, from the voltage change pattern storing means and generates a high-voltage alternating current of which a voltage varies in accordance with the voltage change pattern.

According to the second aspect of the present invention, an electric potential therapy apparatus for generating a negative electric field by applying a high-voltage alternating current that is biased to a negative side, to an electric field generating plate electrode, includes: a waveform change pattern storing means for storing a plurality of waveform change patterns on which a waveform is changed with time; a waveform change pattern selecting means for selecting one from the plurality of waveform change patterns stored in the waveform change pattern storing means; and an alternating current generating circuit which reads the waveform change pattern selected by the waveform change pattern selecting means, from the waveform change pattern storing means and generates a high-voltage alternating current of which a waveform varies in accordance with the waveform change pattern.

According to the third aspect of the present invention, an electric potential therapy apparatus for generating a negative electric field by applying a high-voltage alternating current that is biased to a negative side, to an electric field generating plate electrode, includes: a frequency change pattern storing means for storing a plurality of frequency change patterns on which a frequency is changed with time; a frequency change pattern selecting means for selecting one from the plurality of frequency change patterns stored in the frequency change pattern storing means; and an alternating current generating circuit which reads the frequency change pattern selected by the frequency change pattern selecting means, from the frequency change pattern storing means and generates a high-voltage alternating current of which a frequency varies in accordance with the frequency change pattern.

According to the fourth aspect of the present invention, an electric potential therapy apparatus for generating a negative electric field by applying a high-voltage alternating current that is biased to a negative side, to an electric field generating plate electrode, includes: an alternating current pattern storing means for storing a plurality of alternating current patterns each of which is prepared by combination of a voltage, a waveform and a frequency, each of which is changing with time; an alternating current pattern selecting means for selecting one from the plurality of alternating current patterns stored in the alternating current pattern storing means; and an alternating current generating circuit which reads the alternating current pattern selected by the alternating current pattern selecting means, from the alternating current pattern storing means and generates a high-voltage alternating current of which a voltage, a waveform and a frequency vary in accordance with the alternating current change pattern.

The fifth aspect of the present invention is characterized in that, in the above first and fourth aspects, the voltage change patterns or the alternating current patterns are not of a rectangular wave pattern.

The sixth aspect of the present invention is characterized in that, in the above fifth aspect, for the voltage change patterns or the alternating current patterns, the switching of voltage is implemented by increase or decrease of voltage at steps of 90 to 110 V per second.

The seventh aspect of the present invention is characterized in that, in the above first to sixth aspects, a massage unit is provided.

The eighth aspect of the present invention is characterized in that, in the above seventh aspect, the massage unit includes kneading rollers moving up and down in a backrest and a footrest having a roller massager.

The ninth aspect of the present invention is characterized in that, in the above first to eighth aspects, an ion generator is provided.

### Brief Description of Drawings

FIG. 1 is a side view showing an electric potential therapy apparatus in accordance with the embodiment of the present invention.
FIG. 2 is a block diagram showing an electric configuration of an electric potential therapy apparatus according to the embodiment of the present invention.
FIG. 3 is a chart showing an a.c. waveform that changes with the passage of time in a program.
FIG. 4 is a chart showing an a.c. waveform that changes with the passage of time in a program.
FIG. 5 is a chart showing an a. c. waveform that changes with the passage of time in a program.
FIG. 6 is a chart showing an a. c. waveform that changes with the passage of time in a program.
FIG. 7 is an illustrative chart showing the relationship between a.c. waveforms and frequencies changing with the passage of time in a program.
FIG. 8 is a graph showing the varying output voltage in one cycle in a program P1.
FIG. 9 is an illustrative chart showing the relationship of voltages, waveforms and frequencies with respect to time in one cycle (output voltage cycle) in the program P1.
FIG. 10 is a graph showing the varying output voltage in one cycle in a program P2.
FIG. 11 is a graph showing the varying output voltage in one cycle in a program P3.
FIG. 12 is a graph showing the varying output voltage in one cycle in a program P4.
FIGS. 13 (a) and 13 (b) are graphs of variational examples showing the varying output voltages in one cycle in the program P4.
FIG. 14 is an illustrative chart showing change in a.c. waveform and frequency with the passage of time in the second embodiment.
FIG. 15 an illustrative chart showing change in a.c. waveform and frequency with the passage of time in the second embodiment.
FIG. 16 is an illustrative chart showing change in a. c. waveform and frequency with the passage of time in the third embodiment.
FIG. 17 is a side view showing an electric potential therapy apparatus according to the fourth embodiment with a view of the interior.
FIG. 18 is a front view of FIG. 17.
FIG. 19 a graph showing change in output voltage in a conventional configuration.

### Best Mode for Carrying Out the Invention

### (The first embodiment)

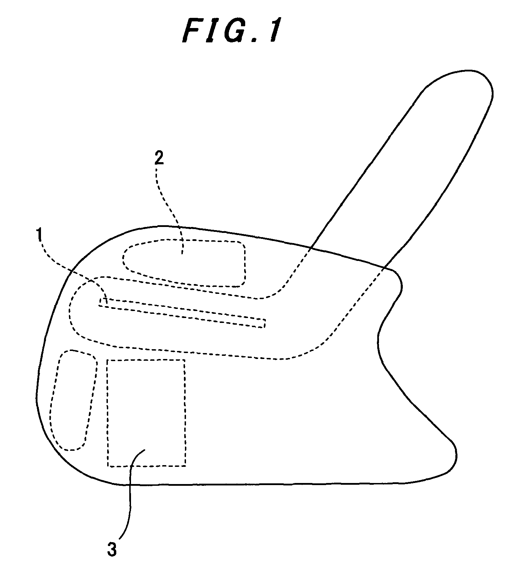

Next, the first embodiment of the present invention will be described with reference to the drawings. FIG. 1 is a side view schematically showing an electric potential therapy apparatus according to the present embodiment. In this figure, 1 designates a plate electrode for generating an electric field and is disposed inside a seat. This electric potential therapy apparatus is controlled by a remote controller 2 removably accommodated in an armrest. Designated at 3 is a main electric potential therapy apparatus including a control circuit for controlling plate electrode 1.

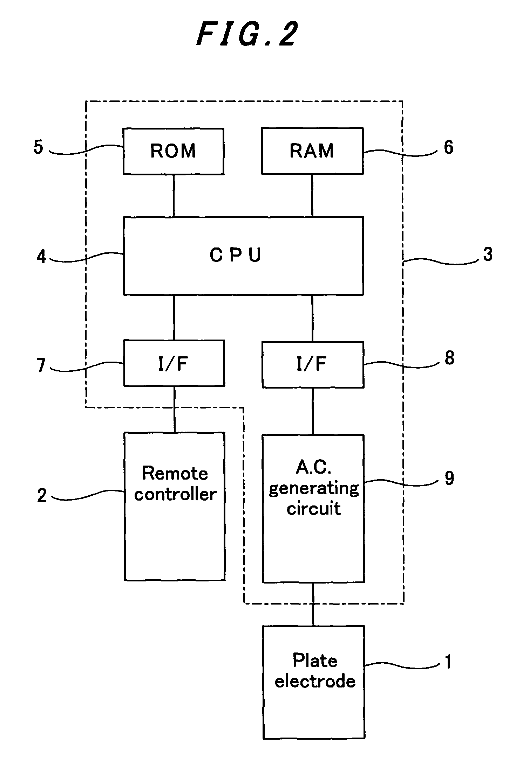

FIG. 2 is a block diagram showing the electric configuration of the electric potential therapy apparatus according to the present embodiment.

Electric potential therapy apparatus 3 includes a microcomputer comprising, mainly, a CPU (central processing unit) 4, a ROM (read-only memory) 5 and a RAM (random-access memory) 6.

A command from remote controller 2 is input into CPU 4 via an interface 7. An output from CPU 4 is sent by way of an interface 8 to an a.c. generating circuit 9 made up of an output transformer and others. An a.c. high voltage generated by a.c. generating circuit 9 is sent to plate electrode 1. The high voltage herein has a magnitude that enables plate electrode 1 to produce an electric field suitable for therapy of a user of the electric potential therapy apparatus, and preferably ranges from 800 V to 9,000 V. On the other hand, its current depends on the distance from plate electrode 1 to the user, the material residing between electrode 1 and the user, and other factors, but is a weak current of some hundreds of microamperes or lower when 9,000 V is applied.

In the present embodiment, a plurality kinds of programs, each of which produces combinations of voltages, a.c. waveforms and frequencies switching with the passage of time, have been prepared in the aforementioned microcomputer, and one program is selected from them by remote controller 2 and activated. The next description hereinbelow will be made referring to a specific example in which four programs are prepared.

### (Waveforms)

To begin with, a plurality kinds of a.c. waveforms used in each program will be described.

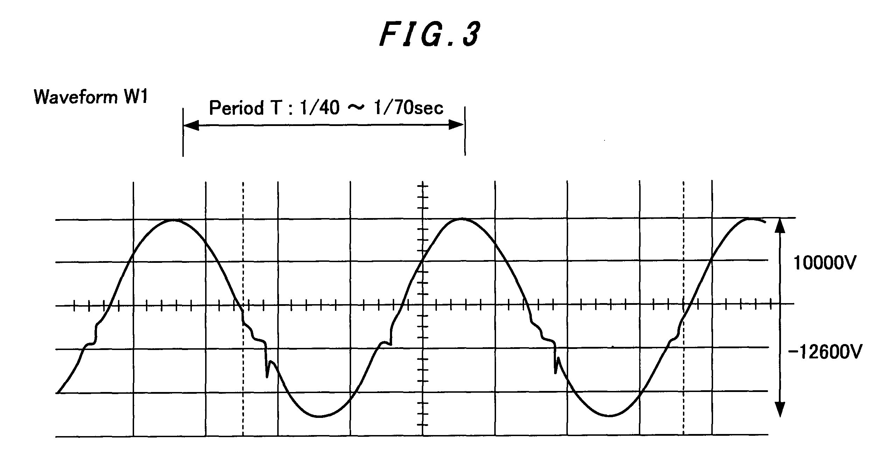

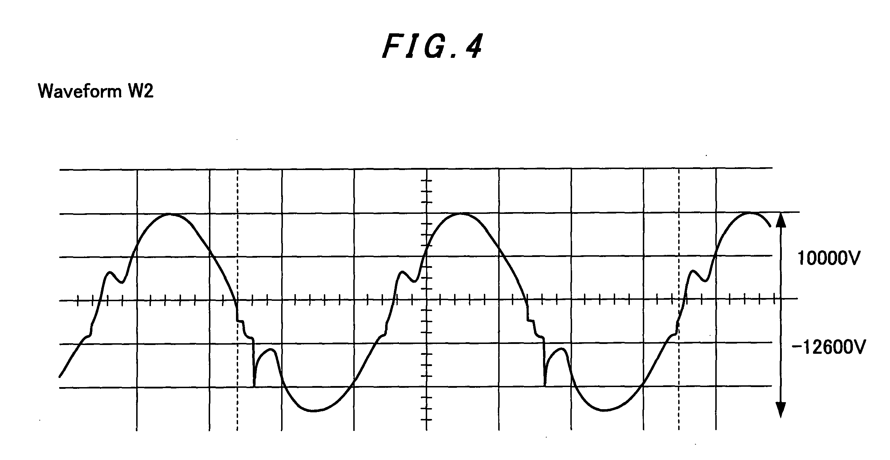

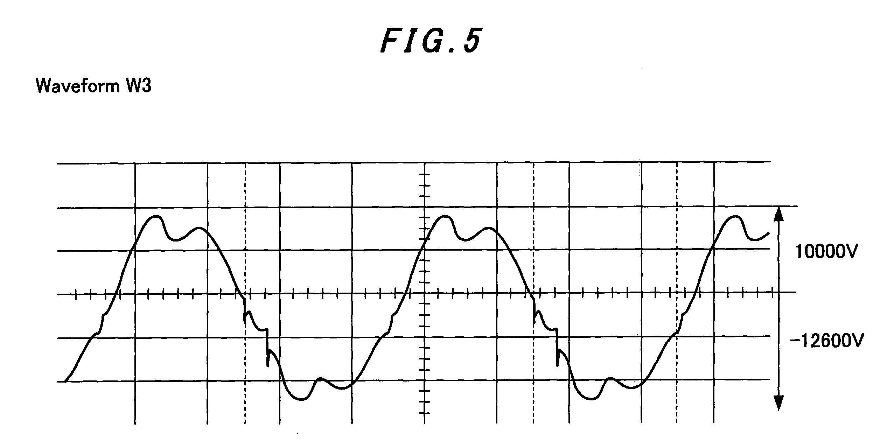

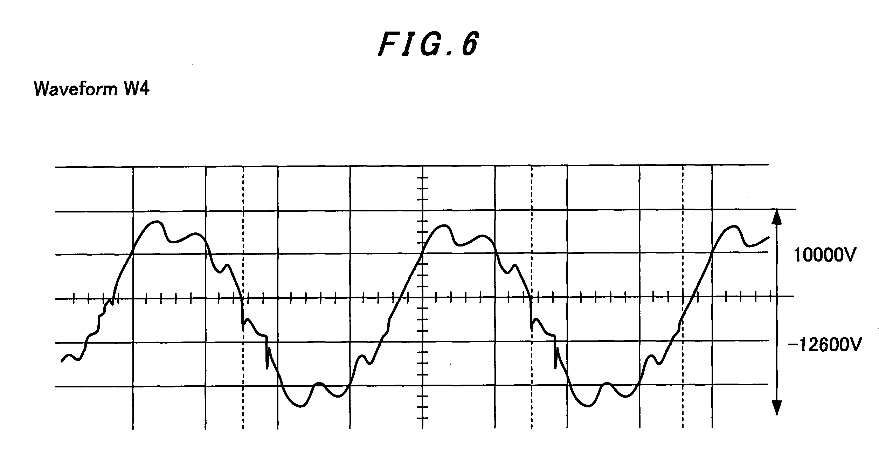

In the present embodiment, for example four kinds of waveforms W1 to W4 are prepared. FIGS. 3 to 6 show waveforms W1 to W4, respectively. Each waveform has a different behavior with the passage of time.

Here, shown in FIGS. 3 to 6, in order to make the difference between waveforms W1 to W4 distinct, is a case where all the waveforms have the same frequency with peak voltages of 10, 000 V and -12, 600 V (a. c. voltage biased to the negative side) and the efficient value of the maximum voltage is 9,000 V.

Waveforms W1 to W4 may and should be different one from another. For example, on the basis of waveform W1, each of waveform W2, waveform W3 and waveform W4 is set up so that the number, timing or magnitudes of crests or trough in one period differ from those of the others. Though the waveforms shown in FIGS. 3 to 6 are formed on the basic waveform of a sine curve, rectangular waves, triangular waves and others may also be used as the basic waveform. That is, variational waveforms can be produced by differentiating the number, timing or magnitudes of crests or troughs in one period from those of the basic waveform. Further, though in the above description, variational waveforms are created based on the same basic waveform, many kinds of variational waveforms can be realized using combinations of different basic waveforms. In such cases, greater undulations of waveforms can be created easily.

Waveforms W1 to W4 are formed by storing waveform data of the shapes of waveforms W1 to W4 into ROM, reading out the stored waveform data and converting it into analog form. Other than this, the waveforms may be generated by electric and electronic circuits.

### (Frequencies)

Next, the frequencies used in each program will be described.

Each of waveforms W1 to W4 has a different frequency from the others so that four different frequencies are used in the present embodiment. For example, as waveform W1 is set at a frequency of 40 Hz, waveform W2 at 50 Hz, waveform W3 at 60 Hz and waveform W4 at 70 Hz, the frequencies of the waveforms are set up a predetermined frequency difference (10 Hz) apart. The frequencies, the frequency difference and the combination of each waveform and each frequency are not limited to the above.

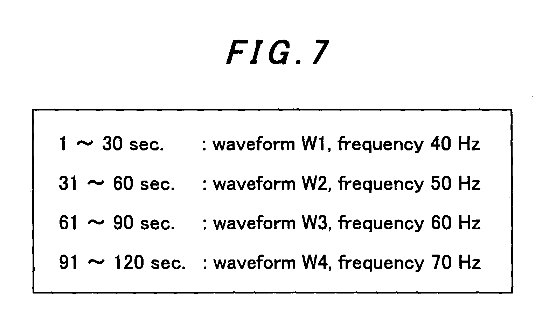

In the present embodiment, waveforms W1 to W4 are switched from one to the next every predetermined period. For example, as shown in FIG. 7, waveforms W1 to W4 are switched sequentially every 30 seconds (waveform continuation time, frequency continuation time), completing one cycle after 2 minutes (waveform cycle, frequency cycle).

### (Output voltages/programs)

Next, output voltages used in each program will be described.

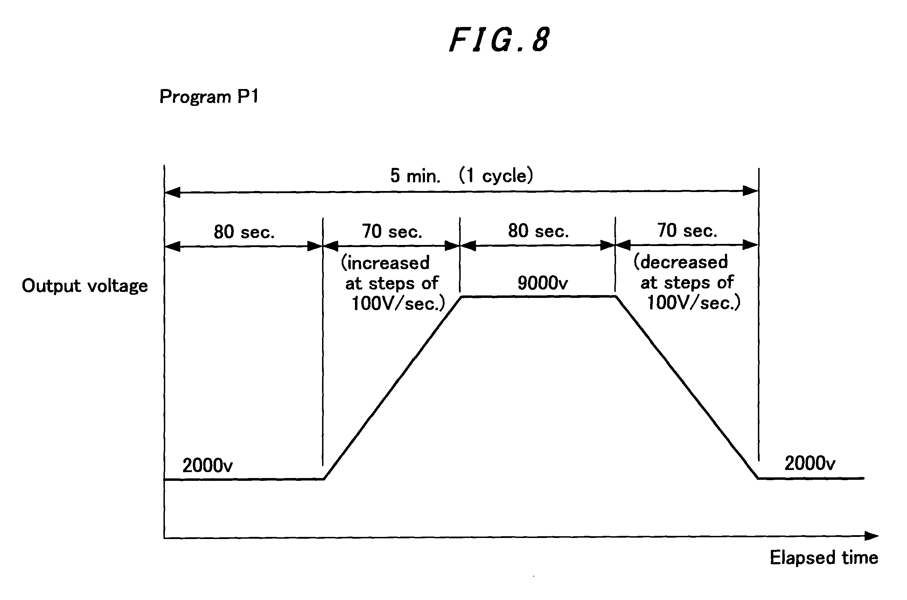

In the present embodiment, four kinds of output voltage patterns VP1 to VP4 are prepared for each program. FIGS. 8, 10, 11 and 12 present patterns of output voltage variation in one cycle (output voltage cycle) in programs P1 to P4, respectively.

### (Output voltage pattern VP1/program P1)

FIG. 8 shows variation in voltage (output voltage pattern VP1) with the passage of time in one cycle (output voltage cycle) in program P1. In this cycle, the output voltage is kept at 2, 000 V for 80 seconds and then is increased a predetermined voltage every predetermined time, for example 100 V every second, up to 9,000 V, in 70 seconds. Next, the voltage is kept at 9,000 V for 80 seconds, then is decreased a predetermined voltage every predetermined time, for example 100 V every second, returned to 2,000 V, in 70 seconds, thus completing one cycle (output voltage cycle). This cycle is repeated 12 times, or for 60 minutes, until the end.

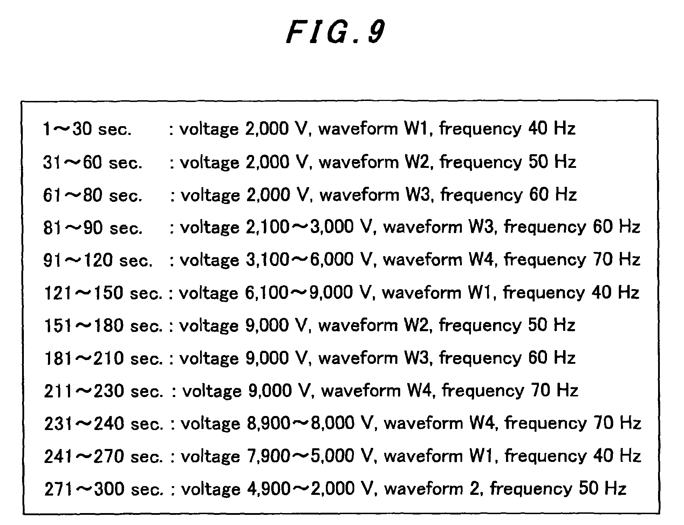

The voltage, waveform and frequency in one cycle (output voltage cycle) in program P1 vary with time, as shown in FIG. 9, for example.

### (Output voltage pattern VP2/program P2)

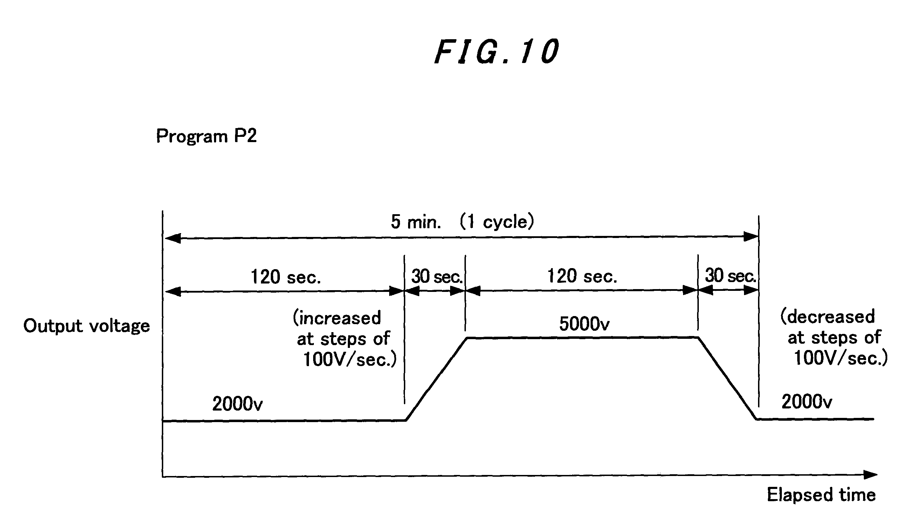

FIG. 10 shows a voltage variation pattern PV2 with the passage of time in one cycle (output voltage cycle) in program P2. In this cycle, the output voltage is kept at 2,000 V for 120 seconds and then is increased a predetermined voltage every predetermined time, for example 100 V every second, up to 5, 000 V, in 30 seconds. Next, the voltage is kept at 5, 000 V for 120 seconds, then is decreased a predetermined voltage every predetermined time, for example 100 V every second, returned to 2, 000 V, in 30 seconds, thus completing one cycle (output voltage cycle). This cycle is repeated 12 times, or for 60 minutes, until the end.

Also in program P2, the output changes sequentially every 30 seconds, in the order of waveform W1 (40 Hz), waveform W2 (50 Hz), waveform W3 (60 Hz) and waveform W4 (70 Hz), together with the above variation in voltage.

### (Output voltage pattern VP3/program P3)

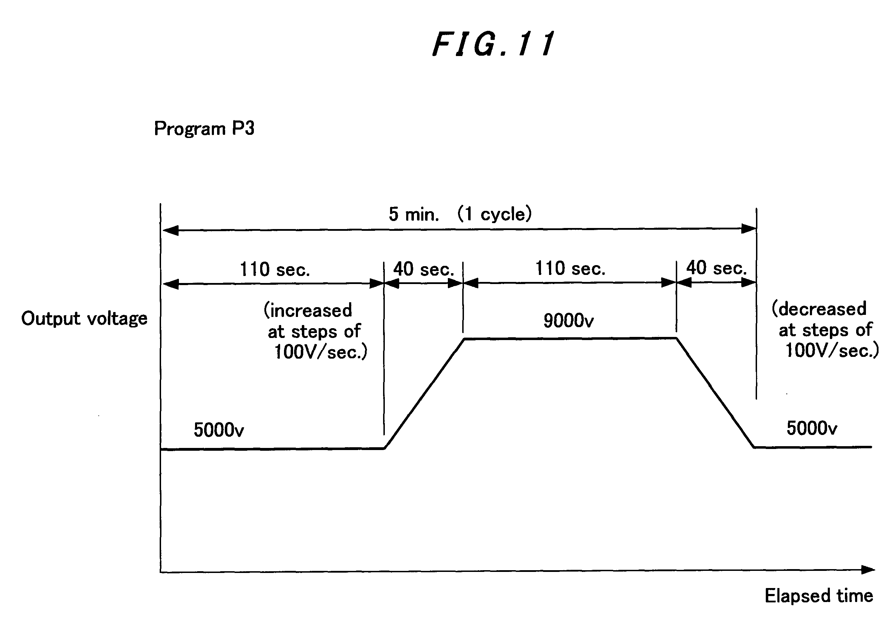

FIG. 11 shows variation in voltage with the passage of time in one cycle (output voltage cycle) in program P3. In this cycle, the output voltage is kept at 5,000 V for 110 seconds and then is increased a predetermined voltage every predetermined time, for example 100 V every second, up to 9,000 V, in 40 seconds. Next, the voltage is kept at 9,000 V for 110 seconds, then is decreased a predetermined voltage every predetermined time, for example 100 V every second, returned to 5,000 V, in 40 seconds, thus completing one cycle (output voltage cycle). This cycle is repeated 12 times, or for 60 minutes, until the end.

Also in program P3, the output changes sequentially every 30 seconds, in the order of waveform W1 (40 Hz), waveform W2 (50 Hz), waveform W3 (60 Hz) and waveform W4 (70 Hz), together with the above variation in voltage.

### (Output voltage pattern VP4/program P4)

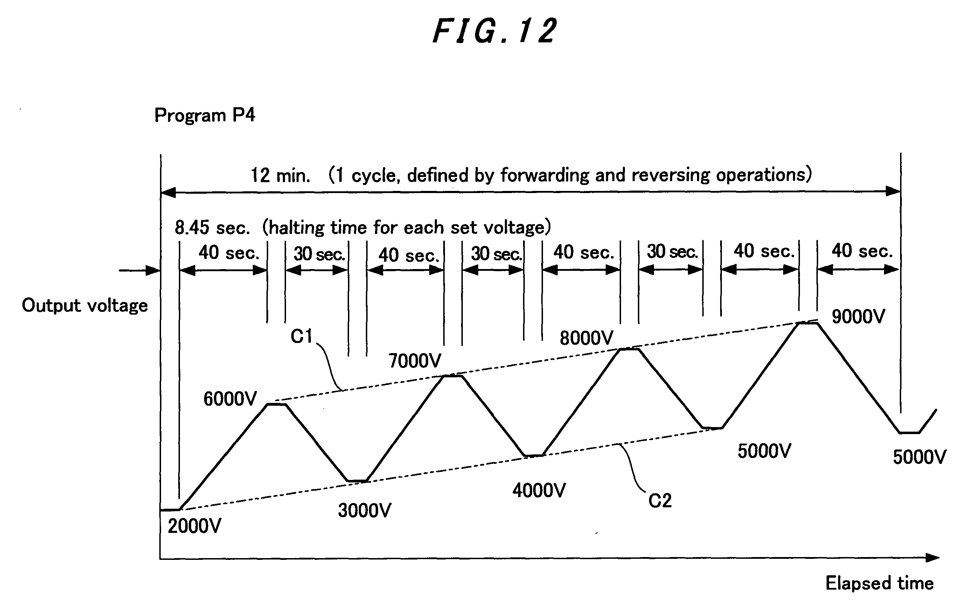

FIG. 12 shows variation in voltage with the passage of time in one cycle (output voltage cycle) in program P4. In this cycle, the output voltage is kept at 2,000 V for 8.45 seconds and then is increased a predetermined voltage every predetermined time, for example 100 V every second, up to 6,000 V, in 40 seconds. Next, the voltage is kept at 6,000 V for 8.45 seconds, then is decreased a predetermined voltage every predetermined time, for example 100 V every second, returned to 3, 000 V, in 30 seconds. Thereafter, similarly to the above, the output voltage is increased to 7,000 V and decreased to 4,000 V, then increased to 8,000 V and decreased to 5000 V, and again increased to 9, 000 V and decreased to 5000 V. This part, up to this point, is half of one cycle, the remaining half is such that the voltage is varied in reverse returning to 2,000 V after increase again to 6,000 V, completing one cycle (output voltage cycle). This cycle is repeated five times or for 60 minutes until the end.

In sum, in program P4, the output voltage is increased and decreased alternately in one cycle (output voltage cycle) and the adjacent crests and adjacent troughs can both take different voltage values. In this way, the voltage of one crest and that of the successive crest are made to change, likewise the voltage of one trough and that of the successive trough, merely by avoiding the adjacent crests being equi-potential as well as avoiding the voltages of the adjacent troughs being so it is possible to increase variation in potential in the therapy pattern, and hence prevent conditioning.

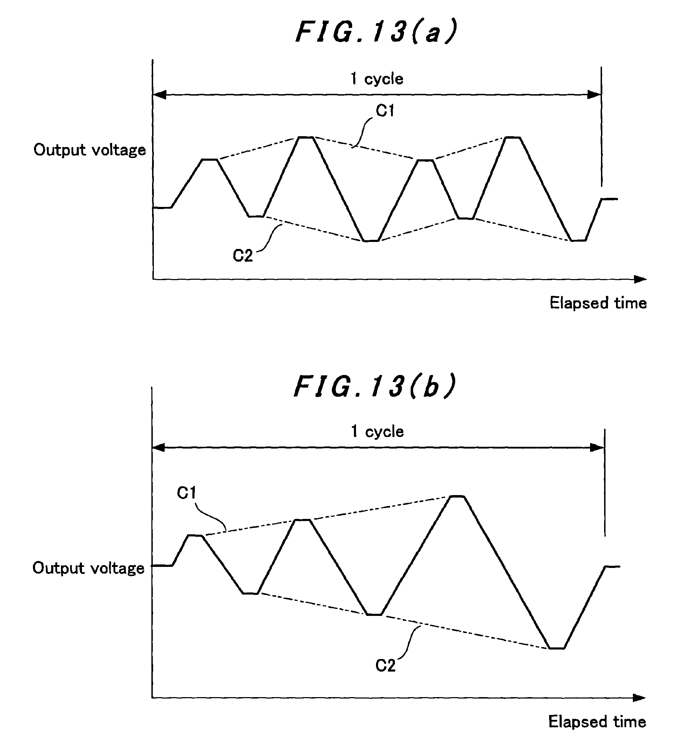

Here, in the pattern shown in this embodiment, both the locus C1 of the potentials of successive crests with the passage of time and the locus C2 of the potentials of successive troughs with the passage of time in one cycle (output voltage cycle) increase gradually (FIG. 12). There are other possible cases such as a pattern in which both the locus C1 of the potentials of successive crests with the passage of time and the locus C2 of the potentials of successive troughs with the passage of time decrease gradually, a case where a potential waveform is formed so that the locus C1 of the potentials of successive crests with the passage of time and the locus C2 of the potentials of successive troughs with the passage of time repeat by themselves up and down in one cycle (output voltage cycle) (FIG. 13 (a)), a case where a potential waveform is formed so that one of the locus C1 of the potentials of successive crests and the locus C2 of the potentials of successive troughs gradually increases while the other gradually decreases (FIG. 13(b)), thus it is possible to increase the number of therapy patterns by potential variation, by creating combinations using various loci C1 of the potentials of successive crests and loci C2 of the potentials of successive troughs, and hence prevent conditioning.

Also in program P4, the output changes sequentially every 30 seconds, in the order of waveform W1 (40 Hz), waveform W2 (50 Hz), waveform W3 (60 Hz) and waveform W4 (70 Hz), together with the above variation in voltage.

As has been described heretofore, in programs P1 to P4 for presenting different output voltage patterns VP1 to VP4 respectively, the output voltage periodically changes its waveform from waveforms W1 to W4 and frequency sequentially in the order of 40 Hz, 50 Hz, 60 Hz and 70 Hz every 2 minutes of one cycle (output voltage cycle). In this way, four types of programs P1 to P4 which each change periodically in output voltage pattern, frequency and a.c. waveform are provided and it is possible for the users to have therapy by selecting the most preferable program from these by considering their own physical condition, physical constitution and so on. Here, if the user felt that the selected program does not suit them, the user is able to stop it and select another one that is suitable.

Further, in the above-described programs, the output voltage waveforms used in programs P1 to P3 (FIGS. 8, 10 and 11) are not rectangular (square or rectangular) waves that take two values, a low voltage (2,000 V, 5,000 V) and a high voltage (9,000 V), only, but are ones that gradually increase or decrease their potential with the passage of time (by varying linearly, in a curved manner, in a triangle-wave like manner, sinusoidally, and so on) at a gentile rate of less than 1,000 V per second, for example. Accordingly, it is possible to avoid causing the user to suffer from stress which would be exerted when a sharp change in potential over 1,000 V or greater (2, 000 V to 9, 000 V, 5, 000 V to 9, 000 V) is applied to the human body. Similarly, the output voltage waveform in program P4 (FIG. 12) does not have a rectangular waveform, is varied from a trough to the adjacent crest (linearly, in a curved manner, in a triangle-wave like manner, sinusoidally, and so on) with the passage of time at a gentile rate of less than, for example 1,000 V per second, whereby it is possible to avoid causing the user to suffer from stress which would be exerted when a sharp change in potential over approximately 1,000 V or greater (2,000 V to 9,000 V, 5,000 V to 9,000 V) is applied to the human body.

Further, increase or decrease of the voltage about 90 to 110 V, preferably 100 V (including approximately 100 V) every second, is able to alleviate the burden on the output transformer, hence reducing degradation of the output transformer and also contributing to extension of the life of the transformer proportionally.

Though the above first embodiment was described referring to a configuration in which one waveform corresponds to one frequency, but the present invention is not limited to this. Now, a case where the frequency changes for one waveform will be described.

### [The second embodiment]

In this embodiment, the frequency changes every predetermined time in a continuous duration of time in which the same a.c. waveform is output. Here, the output voltage patterns of one cycle of programs P1 to P4 are the same as those in the first embodiment so that the description is omitted.

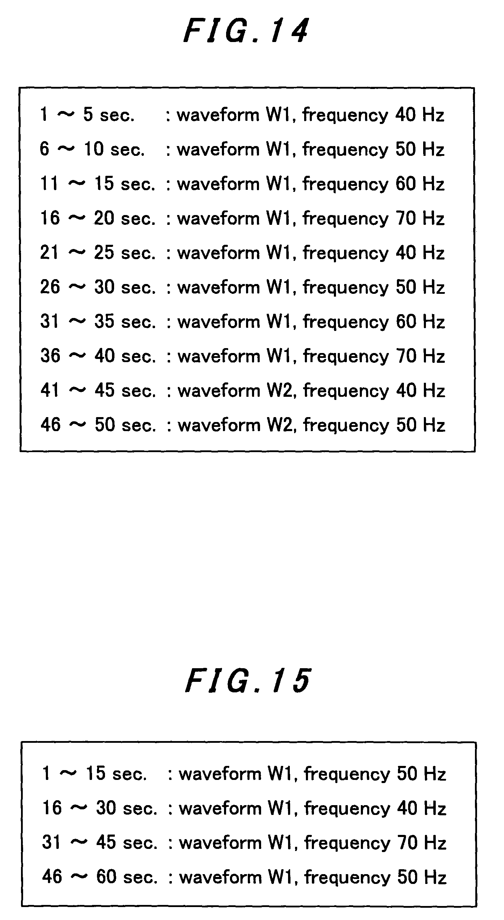

FIG. 14 shows change in a.c. waveform and frequency with the passage of time in the present embodiment.

In this embodiment, waveform W is sequentially changed from one to another every 40 seconds (waveform continuation time), for example, during execution of one program while the frequency is changed every 5 seconds (frequency continuation time),for example, in each waveform continuation time.

The frequency is changed in ascending order with preselected frequencies (40 Hz, 50 Hz, 60 Hz and 70 Hz) and when reaching the maximum value, it returns to the minimum value. Here, the preselected frequencies may be set up by directly defining their values or may be set up by a means that defines the frequencies based on the frequency range and a differential frequency; that is, the setup method should not be limited.

It is also noted that in the present embodiment change of the frequency in the waveform continuation time is set up stepwise in ascending order, but it may be done in descending order or may be done by alternating increase and decrease of the frequency as shown in FIG. 15 (frequency continuation time = 15 seconds).

Also in the second embodiment explained heretofore, the same operational advantage as that in the first embodiment can be obtained.

In the above second embodiment, as an example of the varying frequency for one waveform, a case where the frequency changes periodically in the continuous duration of the same waveform (waveform continuation time) was explained. Another example will be described next.

### [The third embodiment]

In this embodiment, as a configuration of the varying frequency for one waveform, a case where the frequency changes every one cycle (waveform cycle) of each of waveforms W1 to W4 will be described. Here, the output voltage of one cycle of each of programs P1 to P4 is the same as that in the first embodiment so that the description is omitted.

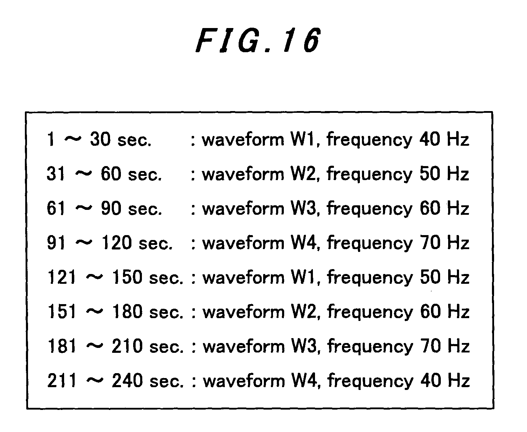

The waveform changes from one to another as shown in FIG. 16, for example every 30 seconds (waveform switching cycle) forming one cycle (waveform cycle) of 2 minutes, and successive waveform cycles are adapted to have different combinations of waveform and frequency. For example, the frequency of the same waveform changes in ascending order among the prepared frequencies, every waveform cycle, and when reaching the maximum value, it returns to the minimum value.

Also, the frequency of the same waveform may change in descending order among the prepared frequencies, every waveform cycle, or the frequency may be increased and decreased in an alternating manner.

The above second and third embodiments were explained taking examples of configurations where the frequency is changed on the basis of each waveform W, but the waveform W may be changed on a frequency basis, in a similar manner.

Also, the combinations of voltage, waveform and frequency are not limited to the above-described four programs, but programs of any other combinations can be used.

Also, more versatile control can be done by providing a configuration where a differential frequency for each waveform is selected.

In addition, in the above programs, the frequency is changed stepwise (non-linearly) but may be changed continuously (linearly).

### [The fourth embodiment]

Next, one embodiment of the above-described electric potential therapy apparatus equipped with a massage machine and an ion generator will be described. Here, the same components as in the above configuration are allotted with the same reference numerals and the description is omitted.

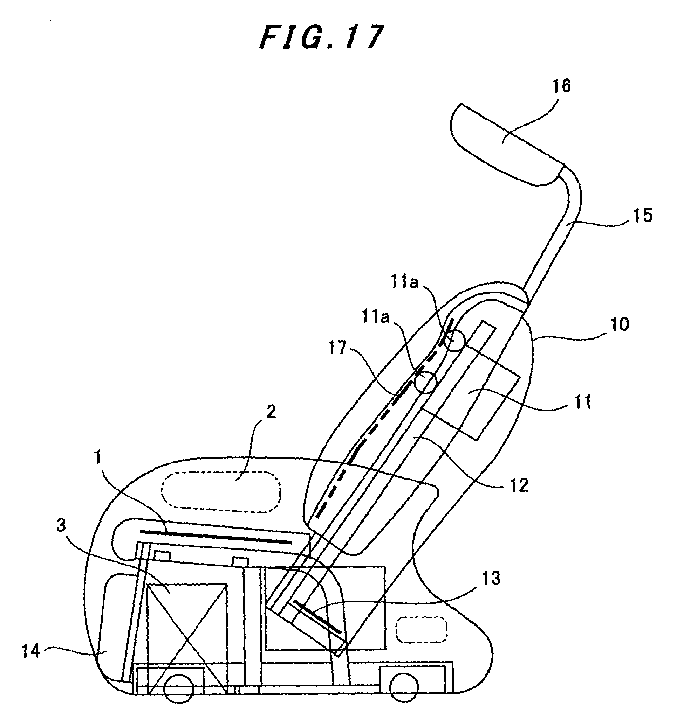

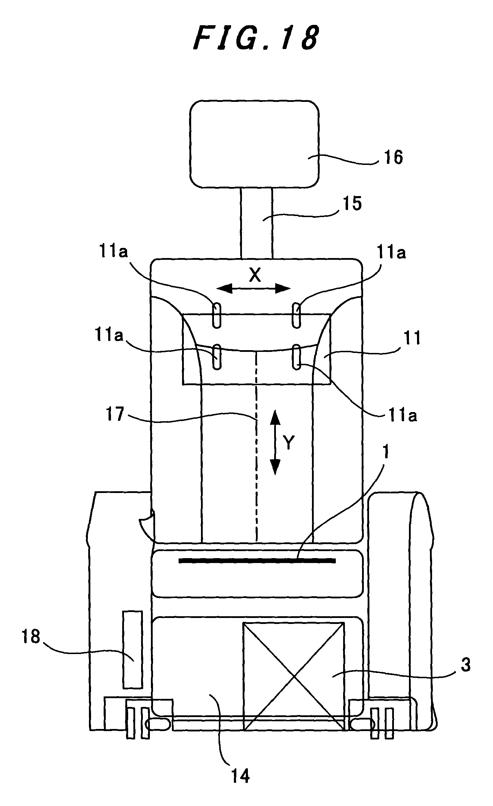

FIG. 17 is a side view showing the interior and FIG. 18 is the front view of FIG. 17. A massage unit 11 provided inside a backrest 10 is a massage machine which massages the neck and shoulders, back and waist area, by applying pressure and/or holding and shifting the massage section with the passage of time. As shown in FIG. 18, the massage unit includes in total four kneading rollers 11a, two rollers arranged a predetermined distance apart with respect to the width direction X of backrest 10 and two rows of such rollers arranged a predetermined distance apart with respect to the length direction Y (in the height direction of the user to be massaged).

Massage unit 11 can be moved up and down, guided by a rail 12 extending in the longitudinal direction Y, by means of an undescribed up/down motor, so that kneading rollers 11a can move from the shoulder position to the waist position. Kneading rollers 11a arranged in the width direction X are allowed to move in opposite directions with respect to the width X, with the passage of time, so that their pressure points and the kneading area held and pressed between two kneading rollers 11a can be changed with time. In addition, it is also possible to administer a holding and compressing massage in the longitudinal direction Y by permitting kneading rollers 11a in the longitudinal direction Y to move in opposite directions with respect to the longitudinal direction Y, with time.

A control board 13 for massage unit 11 is disposed close to the bottom end of rail 12.

A footrest 14 includes a rotary type massager made up of rollers etc., which enable pressing and holding and compressing of user's feet, e.g., soles of feet (including soles and side areas) and calves, changing the massage points with time, and is arranged in front of the seat incorporating electrode 1.

Footrest 14 is constructed so that the rotary type massager can be changed in position in accordance with the points to be massaged. That is, the footrest is constructed so as to pivot or move forward or in other ways so that the rotary massager is positioned at the lower position in front of the seat when used for sole massage purposes and is located at the position approximately extended from the seat top when used for calf massage purposes.

A support bar 15 is arranged at the top of backrest 10 and supports an ion generating unit 16 at its distal end. Ion generating unit 16 emits negative ions in an amount of approximately 1,200,000 ions/cc toward backrest 10 and thereabout. Negative ions neutralize positive ions generated from electronic appliances, etc., presenting a forest bath effect, refreshing effect, to promote recovery from exhaustion.

An electric heater 17 is disposed underneath the surface of backrest 10. The main electric potential therapy apparatus 3, massage unit 11, footrest 14, ion generating unit 16 and electric heater 17 are controlled by a remote controller 2 which is removably accommodated in the armrest. A main control board 18 is arranged inside the right armrest, and switches between the electric potential therapy and the massage function. This electric potential therapy apparatus has a reclining function, so that backrest 11 leans back while footrest 14 moves (advances) in the direction in which the angle of footrest 14 with the seat becomes smaller (in the direction in which the footrest is positioned approximately flush with the seat).

With this arrangement, when using this electric potential therapy apparatus in the electric potential therapy mode, the user first gets seated on the electric potential therapy apparatus and controls remote controller 2 to activate the main electric potential therapy apparatus 3, whereby high voltage is generated and applied to electrode 1, and use is made for electric potential therapy purposes. When the apparatus is used as a massager, after getting seated on the electric potential therapy apparatus, the user controls remote controller 2 to activate massage unit 11 and also activate the rotary massager inside footrest 14, so that use is made for massager purposes. When the apparatus is used as an ion generator, the user controls remote controller 2 to turn on the power to ion generating unit 16, whereby ion generating unit 16 generates negative ions. When the user wants to warm their back, the user may control remote controller 2 to turn on the switch of electric heater 17.

As has been described, with only a single electric potential therapy apparatus it is possible to give electric potential therapy to the user, who has been recovered from muscle fatigue and is improved in blood circulation and is relaxed in mind and body by administration of massage under the environment that can provide a forest effect and refreshing effect by negative ions, or it is possible to administer a massage in the condition after an electric potential therapy, whereby it is possible to produce a synergy effect. In this way, execution of an electric potential therapy with use of massagers is more effective compared to the case where an electric potential therapy and a massage treatment are individually effected.

Further, a user who needs an electric potential therapy apparatus and a massager, an electric potential therapy apparatus and an ion generator, or an electric potential therapy apparatus, a massager and an ion generator, does not need to buy them individually, but all the user need to do is to purchase this single apparatus. Thus, it is possible to reduce the area of installation to half or lower compared to the case where all the conventional dedicated appliances are installed.

As has been described, according to the present invention, a plurality of programs having different intensities can be prepared by combinations of changing voltages, waveforms and frequencies with the passage of time, so as to allow the user to select one program from them, which is deemed to have the most suitable intensity, and actuate the electric potential therapy apparatus in the selected mode. Therefore, the user is able to receive an optimal therapy which meets the user's physical constitution, physical condition and the number of times of use of the user and so on.

Since no rectangular wave pattern is used for the a.c. patterns presenting varying voltage with the passage of time, it is possible to avoid the user from receiving stress which would be caused when a sharp change in potential is applied.

Since switch of voltage based on a.c. patterns is implemented by increasing or decreasing voltage at steps of 90 to 110 V per second, it is possible to alleviate the burden on the output transformer, hence reducing degradation of the output transformer and also extending its life proportionally.

Integration of a massager and an ion generator with an electric potential therapy apparatus enables the user to make a total recovery and refreshment from exhaustion using a single electric potential therapy apparatus. Further, a user who needs an electric potential therapy apparatus and a massager, an electric potential therapy apparatus and an ion generator, or an electric potential therapy apparatus, a massager and an ion generator, does not need to buy them individually, but all the user need to do is to purchase this single apparatus. Thus, it is possible to reduce the area of installation to half or lower compared to the case where all the conventional dedicated appliances are installed.

### Industrial Applicability

The electric potential therapy apparatus according to the present invention is suitably used for administration of therapy for stiff shoulders, headache, insomnia, chronic constipation, etc., which meets the user's physical constitution, physical condition and the number of times of use of the user and so on. Further, the present apparatus is suitably applied to an electric potential therapy apparatus which also administrates massages and provides a forest effect and a refreshing effect by negative ions.

## Claims

1. An electric potential therapy apparatus for generating a negative electric field by applying a high-voltage alternating current that is biased to a negative side, to an electric field generating plate electrode, comprising:
a voltage change pattern storing means for storing a plurality of voltage change patterns on which a voltage is changed with time;
a voltage change pattern selecting means for selecting one from the plurality of voltage change patterns stored in the voltage change pattern storing means; and
an alternating current generating circuit which reads the voltage change pattern selected by the voltage change pattern selecting means, from the voltage change pattern storing means and generates a high-voltage alternating current of which a voltage varies in accordance with the voltage change pattern.

2. An electric potential therapy apparatus for generating a negative electric field by applying a high-voltage alternating current that is biased to a negative side, to an electric field generating plate electrode, comprising:
a waveform change pattern storing means for storing a plurality of waveform change patterns on which a waveform is changed with time;
a waveform change pattern selecting means for selecting one from the plurality of waveform change patterns stored in the waveform change pattern storing means; and
an alternating current generating circuit which reads the waveform change pattern selected by the waveform change pattern selecting means, from the waveform change pattern storing means and generates a high-voltage alternating current of which a waveform varies in accordance with the waveform change pattern.

3. An electric potential therapy apparatus for generating a negative electric field by applying a high-voltage alternating current that is biased to a negative side, to an electric field generating plate electrode, comprising:
a frequency change pattern storing means for storing a plurality of frequency change patterns on which a frequency is changed with time;
a frequency change pattern selecting means for selecting one from the plurality of frequency change patterns stored in the frequency change pattern storing means; and
an alternating current generating circuit which reads the frequency change pattern selected by the frequency change pattern selecting means, from the frequency change pattern storing means and generates a high-voltage alternating current of which a frequency varies in accordance with the frequency change pattern.

4. An electric potential therapy apparatus for generating a negative electric field by applying a high-voltage alternating current that is biased to a negative side, to an electric field generating plate electrode, comprising:
an alternating current pattern storing means for storing a plurality of alternating current patterns each of which is prepared by combination of a voltage, a waveform and a frequency, each of which is changing with time;
an alternating current pattern selecting means for selecting one from the plurality of alternating current patterns stored in the alternating current pattern storing means; and
an alternating current generating circuit which reads the alternating current pattern selected by the alternating current pattern selecting means, from the alternating current pattern storing means and generates a high-voltage alternating current of which a voltage, a waveform and a frequency vary in accordance with the alternating current change pattern.

5. The electric potential therapy apparatus according to Claim 1, wherein the voltage change patterns are not of a rectangular wave pattern.

6. The electric potential therapy apparatus according to Claim 4, wherein the alternating current patterns are not of a rectangular wave pattern.

7. The electric potential therapy apparatus according to Claim 5 or 6, wherein the switching of voltage is implemented by increase or decrease of voltage at steps of 90 to 110 V per second.

8. The electric potential therapy apparatus according to Claims 1 to 7, further comprising a massage unit.

9. The electric potential therapy apparatus according to Claim 8, wherein the massage unit includes kneading rollers moving up and down in a backrest and a footrest having a roller massager.

10. The electric potential therapy apparatus according to Claims 1 to 9, further comprising an ion generator.
